Europäisches Patentamt

European Patent Office

Office européen des brevets

(19)

(11) Numéro de publication: **0 209 456 B1**

# FASCICULE DE BREVET EUROPEEN

(12)

(45) Date de publication du fascicule du brevet: **16.01.91**

(51) Int. Cl.⁵: **C 07 D 457/02**

(21) Numéro de dépôt: **86401539.1**

(22) Date de dépôt: **10.07.86**

(54) Procédé de préparation du N-méthyl lysergol.

(30) Priorité: **11.07.85 FR 8510621**

(43) Date de publication de la demande:
**21.01.87 Bulletin 87/04**

(45) Mention de la délivrance du brevet:
**16.01.91 Bulletin 91/03**

(84) Etats contractants désignés:
**AT BE CH DE FR GB IT LI LU NL SE**

(56) Documents cités:
**EP-A-0 004 664**
**FR-A-1 440 188**

**SYNTHESIS, 1972, page 566, Stuttgart, DE; G.M. RUBOTTOM: "The alkylation of indole sodium salt"**

Le dossier contient des informations techniques présentées postérieurement au dépôt de la demande et ne figurant pas dans le présent fascicule.
(73) Titulaire: **RHONE-POULENC SANTE**
**20, avenue Raymond Aron**
**F-92160 Antony (FR)**

(72) Inventeur: **Gervais, Christian**
**6 allée Marchel Achard**
**F-69100 Villeurbanne (FR)**

(74) Mandataire: **Pilard, Jacques et al**
**RHONE-POULENC RECHERCHES Service**
**Brevets Pharma 25, Quai Paul Doumer**
**F-92408 Courbevoie Cedex (FR)**

Courier Press, Leamington Spa, England.

# Description

La présente invention concerne un nouveau procédé de préparation du méthyl-1 lysergol qui est un intermédiaire particulièrement intéressant dans la synthèse de produits à activité pharmacologique qui sont décrits dans le brevet américain US 3 228 943.

Il est connu, en particulier d'après le brevet français FR 79 08608 publié sous le numéro 2 421 901 de préparer le méthoxy-10α méthyl-1 lumilysergol par action de l'iodure de méthyle en présence de potasse en opérant dans le diméthyl-sulfoxyde. Cependant dans ces conditions il y a compétition entre la méthylation de l'atome d'azote indolique et celle de la fonction alcool primaire.

R. M. Rubottom et al, Synthesis, p. 556 (1972) enseignent la méthylation de dérivés de l'indole au moyen d'iodure de méthyle en présence d'hydrure de sodium en opération dans l'hexaméthyl-phosphotriamide. Cependant, ces conditions ne donnent pas des résultats satisfaisants lorsqu'elles sont appliquées à la méthylation du lysergol.

Il a maintenant été trouvé, et c'est ce qui fait l'objet de la présente invention, que la méthylation du lysergol peut être réalisée sélectivement en ajoutant une base choisie parmi les hydrures et les alcoolates de métaux alcalins à une solution de lysergol dans un solvant aprotique polaire, de telle manière que le rapport moléculaire entre la base et le lysergol reste inférieur à 0,5, puis un agent de méthylation choisi parmi l'iodure de méthyle, le sulfate de méthyle et l'iodure de triméthylsulfonium à une vitesse telle que celui-ci ne soit pas en excès par rapport à la base pour éviter la formation de dérivés ammonium quaternaires.

Les solvants aprotiques polaires qui conviennent particulièrement bien sont la N-méthylpyrrolidone, l'hexaméthylphosphotriamide, la tétraméthylurée, le diméthylformamide et le diméthylacétamide.

La sélectivité de la réaction est favorisée si la base utilisée est choisie parmi les hydrures de sodium et de potassium.

Généralement, le procédé selon l'invention est mis en oeuvre à une température comprise entre 0 et 60°C.

Le méthyl-1 lysergol obtenu par la mise en oeuvre du procédé est séparé et isolé selon les méthodes habituelles.

# Revendications

1. Procédé de préparation du méthyl-1 lysergol caractérisé en ce que l'on ajoute une base choisie parmi les hydrures et les alcoolates de métaux alcalins à une solution de lysergol dans un solvant aprotique polaire, de telle manière que le rapport moléculaire entre la base et le lysergol reste inférieur à 0,5, puis un agent de méthylation choisi parmi l'iodure de méthyle, le sulfate de méthyle et l'iodure de triméthylsulfonium à une vitesse telle que celui-ci n'est pas en excès par rapport à la base.

2. Procédé selon la revendication 1 caractérisé en ce que le solvant aprotique polaire est choisi parmi la N-méthylpyrrolidone, l'hexaméthylphos-photriamide, la tétraméthylurée, le diméthylformamide et le diméthylacétamide.

# Patentansprüche

1. Verfahren zur Herstellung von 1-Methyllysergol, dadurch gekennzeichnet, daß man einer Lösung von Lysergol in einem polaren aprotischen Lösungsmittel eine Base, ausgewählt aus den Alkalimetallhydriden und -alkoholaten, zusetzt, derart, daß das molekulare Verhältnis zwischen Base und Lysergol unterhalb 0,5 bleibt, und dann ein Methylierungsmittel, ausgewählt aus Methyljodid, Methylsulfat und Trimethylsulfoniumjodid, mit einer solchen Geschwindigkeit zusetzt, daß es nicht im Überschuß in bezug auf die Base vorliegt.

2. Verfahren nach Anspruch 1, dadurch gekennzeichnet, daß das polare aprotische Lösungsmittel ausgewählt ist aus N-Methylpyrrolidon, Hexamethylphosphotriamid, Tetramethylharnstoff, Dimethylformamid und Dimethylacetamid.

# Claims

1. A process for preparing 1-methyllysergol, wherein a base, chosen from alkali metal hydrides and alcoholates, is added to a solution of lysergol in a polar aprotic solvent, in such a way that the molecular ratio between the base and the lysergol remains less than 0.5, and a methylating agent chosen from methyl iodide, methyl sulphate and trimethylsulphonium iodide is then added at a rate such that the latter is not in excess with respect to the base.

2. A process according to claim 1, wherein the polar aprotic solvent is chosen from N-methyl-pyrrolidone, hexamethylphosphotriamide, tetra-methylurea, dimethylformamide and dimethyl-acetamide.